(19) **Europäisches Patentamt**

**European Patent Office**

**Office européen des brevets**

(11) **EP 1 309 308 B1**

(12) **EUROPEAN PATENT SPECIFICATION**

(45) Date of publication and mention
of the grant of the patent:
**25.10.2006 Bulletin 2006/43**

(51) Int Cl.:
***A61Q 9/04*** (2006.01)

(21) Application number: **01963123.3**

(22) Date of filing: **09.08.2001**

(86) International application number:
**PCT/GB2001/003580**

(87) International publication number:
**WO 2002/011687 (14.02.2002 Gazette 2002/07)**

(54) **EPILATORY COMPOSITIONS**

ENTHAARUNGSMITTEL

COMPOSITIONS DEPILATOIRES

(84) Designated Contracting States:
**AT BE CH CY DE DK ES FI FR GR IE IT LI LU MC NL PT SE TR**

(30) Priority: **10.08.2000 GB 0019651**

(43) Date of publication of application:
**14.05.2003 Bulletin 2003/20**

(73) Proprietor: **Reckitt Benckiser (UK) Limited Slough,
Berkshire SL1 3UH (GB)**

(72) Inventors:
• **ACHER, David
Beverley,
East Yorkshire HU17 8JL (GB)**
• **DE LA TORRE, Frederic
Beverley,
East Yorkshire HU17 0HN (GB)**

(74) Representative: **Hall, Marina
Reckitt Benckiser plc,
Group Patents Department,
Dansom Lane
Hull HU8 7DS (GB)**

(56) References cited:
**EP-A- 0 236 219        FR-A- 2 656 524
GB-A- 941 081**

• **DATABASE WPI Week 198528 Derwent Publications Ltd., London, GB; AN 1985-168487 XP002187690 & JP 60 097912 A (SHISEIDO)**
• **PATENT ABSTRACTS OF JAPAN vol. 2000, no. 19, 5 June 2001 (2001-06-05) & JP 2001 054422 A (KANEBO ; KUROISUTAA CHEMICALS), 27 February 2001 (2001-02-27)**

Remarks:
The file contains technical information submitted after the application was filed and not included in this specification

**Description**

This invention relates to an epilatory composition, and its use.

Epilatory compositions formed of viscoelastic materials are well known. The viscoelastic materials may in certain embodiments be rosin-based. In other embodiments they may be sugar-based. A tackifier, such as colophony, may be included to make them sticky.

In some products the epilatory compositions may be supplied in the form of strips, retained between cellophane sheets. The cellophane sheets may have coatings of polyvinyl chloride, which acts as a barrier preventing the composition, or components of it, from migrating through the sheets; and also having the correct adhesive properties for use. In use, the user peels away one of the cellophane sheets, presses the epilatory strip firmly onto the area to be plucked, then pulls one end of the remaining cellophane sheet sharply away from the area. The hairs trapped in the composition are removed from the treated area along with, optimally, all of the composition, still attached to the remaining backing strip.

In an alternative approach a composition may be warmed, and then applied to the skin by means of a spatula or other applicator. Strips of fabric are then applied so that they adhere to the epilatory composition. The strips are then pulled sharply to remove the epilatory material, and hair, from the skin.

In both approaches the viscoelastic properties of the compositions are important. However this is particularly so in the case of the compositions supplied as strips, since these are applied to the skin at ambient temperature. At ambient temperature the compositions should be soft and pliable, such that they mould closely to the body shape. On the other hand they must not be so soft that they flow prior to use. When they are in place on the body and the user pulls the remaining backing strip, applying a high frequency strain rate to the compositions, their elastic properties should predominate over their viscous properties.

Epilatory compositions containing rosin have been disclosed in, for example FR-A-2 656 524 and EP-A-0 236 219. However, there is a particular problem with known compositions supplied in the form of strips, in meeting one of the requirements described in the previous paragraph. It is, that under warm ambient conditions the compositions may flow, and leak out from between the sheets. One approach to counter this has been to supply strips with considerably over-sized cellophane sheets. However, clearly, this approach is inadequate as a solution to a situation where there might be a substantial flow of a composition. It is also inefficient in terms of materials and transportation, and undesirable from a marketing perspective, in that consumers perceive that such a product is of poor value.

Accordingly, there is a need for an epilatory composition with improved resistance to flow of the composition, in warm environments, prior to use.

In accordance with a first aspect of the present invention there is provided an epilatory composition comprising a matrix rosinous or sugar-based material in admixture with particulate fumed silica.

The matrix material is suitably a gel-like material with adhesive properties.

The matrix material could be sugar-based. Examples of specifications describing epilatory compositions based on heating sugar(s) with acids (such as citric acid) are disclosed in GB 901624, GB 1242083, GB 2231494A, and GB 2157951A.

Preferably, however, the matrix material comprises a tackifying rosinous material, for example a rosin ester and/or colophony.

Preferably the epilatory composition comprises at least 60% wt/wt of rosinous material, preferably at least 70% wt/wt, more preferably at least 80% wt/wt.

Preferably the epilatory composition is a so-called "cold" epilatory composition (that is, one which can be applied at ambient temperature without reheating).

Preferably the particulate fumed silica has particles of mean diameter 1-200 nm, more preferably 5-100 nm, and most preferably 10-50 nm.

Preferably the particles are present in the matrix material in an amount of at least 1% wt/wt, more preferably at least 2% wt/wt, and most preferably at least 3% wt/wt. Suitably they are present in an amount up to 40% wt/wt, preferably up to 20% wt/wt, and most preferably up to 10% wt/wt.

Fumed silica is currently manufactured in a process that involves flame hydrolysis of silicon tetrachloride, in an oxy-hydrogen flame. It is a colloidal form of silica having silanol groups, able to participate in hydrogen bonding. Fumed silica typically comprises colloidal particles of mean diameter 1-200 nm. Preferably the fumed silica is of mean diameter 5-100 nm, more preferably 10-50 nm. The external surface area is typically in the range 15-380 $m^2$/g. Fumed silicas are typically non-porous and thus have no internal surface area. They may be hydrophobic and of use in the present invention but preferred fumed silicas for use in the present invention are hydrophilic.

The epilatory composition may suitably comprise up to 40%, preferably up to 20%, of other components, which may include one or more of a natural wax, a fragrance, a polymer, an essential oil, a silicone oil, a colorant, an anti-oxidant or a paraffin or mineral oil.

Suitably an epilatory composition comprising a rosinous material, when formed into sheets and not under applied stress, is shape-stable for a period of 6 months at all temperatures in the range 20-50°C.

Suitably an epilatory composition comprising a rosinous material and particulate fumed silica, when formed into sheets and not under applied stress, is shape-stable for a period of 6 months at all temperatures in the range 20-50°C; whereas the corresponding matrix material not containing any said particles, when formed into flat sheets and not under applied stress, flows under its own weight at least some temperatures in the range 20-50°C during a period of 6 months.

Suitably the epilatory composition is such that its elastic modulus exceeds its viscous modulus at all frequencies up to 0.1 rad/s at 50°C.

Preferably the elastic modulus of the epilatory composition exceeds its viscous modulus at all frequencies up to 1 rad/s at 50°C, more preferably at all frequencies up to 2 rad/s at 50°C.

In certain embodiments, notably epilatory compositions having a sugar-based matrix material, the elastic modulus may exceed the viscous modulus at all frequencies up to 20 rad/s at 50°C.

Preferably at certain higher frequencies (representative of the rapid removal of the epilatory composition from the user's skin), the elastic modulus also exceeds the viscous modulus, at temperatures within the temperature range 20-50°C.

Preferably the elastic modulus exceeds the viscous modulus (when measured at 35°C) at a frequency of at least 10,000 rad/s, more preferably at a frequency at least 5,000 rad/s.

Thus, preferably the epilatory composition is such that, at ambient temperatures, at low frequencies of applied stress the elastic modulus exceeds the viscous modulus; at high frequencies of applied stress the elastic modulus exceeds the viscous modulus; and at moderate frequencies, in between, the viscous modulus exceeds the elastic modulus. The epilatory composition in transit and storage corresponds to the low frequency condition, and the non-viscous nature of the composition aids shape stability in storage and transit; the application of the epilatory composition to the skin corresponds to the moderate frequency condition, and the viscous nature of the composition aids application and good contact with hair and skin; and pulling the epilatory composition sharply from the skin corresponds to the high frequency condition, the non-viscous, glassy nature of the composition aiding effective hair removal. The transition between the low frequency condition and the moderate frequency condition is known as the gel point. The transition between the moderate frequency condition and the high strain rate condition is known as the glass transition.

The elastic modulus G' (sometimes known as the storage modulus) corresponds to the energy which can be stored and released by a bulk material. The viscous modulus G" (sometimes known as the loss modulus) corresponds to the energy dissipated by a bulk material due to friction between its macromolecules when it is deformed.

$$G' = \frac{\sigma}{\gamma} \cos \delta$$

$$G'' = \frac{\sigma}{\gamma} \sin \delta$$

wherein σ is the stress amplitude, γ is the strain amplitude and δ is the out-of-phase coefficient.

The measurements quoted later are based on studies carried out into the rheology of the viscoelastic compositions in order to obtain a better understanding of their adhesive behaviour and their suitability as epilatory materials. These studies involved subjecting the materials to dynamic investigations in which a sinusoidal strain at defined frequencies was applied to the materials and the resulting output force was measured. In these studies a stress control rheometer was used, the SR rheometer commercially available from the company Rheometrics, using parallel plate geometry of 25 mm in diameter. The output force was found to include an in-phase elastic component G' and an out-of-phase viscous component G". The output force can be expressed as follows.

$$\sigma = \sigma \sin(t\omega + \delta)$$
$$= \sigma \cos\delta \sin t\omega + \sigma \cos\delta \cos t\omega$$

where ω is the test frequency and t is the time.

Within the linear stress-strain domain of the material G' is desirably lower than G" at moderate frequency oscillation in order to prevent the material cracking and to ensure that the material has strong adhesion at the material/hair interface. The values of G' and G" at moderate frequency oscillation are a measure of how readily the material wets the hairs. Moderate frequency oscillation is a long time process and corresponds to the time when the material is being applied to the skin. The lower values of G' and G" at this moderate frequency, the better the material wets the hairs. Thus the hairs become well embedded in the material in a very short time (ie the time needed for spreading the material on the skin). However G' should be higher than G" at high frequency oscillation (which mimics the action of the user in rapidly pulling the strip from the body) in order to remove hairs efficiently. Also, at low frequency oscillation, or no oscillation, G' is preferably higher than G", in accordance with this invention, in order to obtain the benefit of enhanced stability, even when warm.

The definitions given herein refer to stresses applied to the material within its linear stress-strain domain, which may typically be up to a few thousand Pa.

By ensuring that the epilatory composition satisfies the above parameters, it can be readily applied to the skin at body temperature, yet it is very efficient at removing hairs from the skin and, surprisingly, the user experiences less pain.

References in this specification to a material not under applied stress are to a material in the form of a flat sheet, resting on a horizontal surface.

Whilst we are not bound by any theory, we believe that the particles form a network throughout the epilatory composition, providing a structure or backbone which inhibits the flow of the composition, at warm temperatures.

If wished the epilatory composition of the present invention may be provided in a container, from which the user removes it using, for example, a spatula or an applicator fitted to the container, and applies it to the skin. A fabric can then be used to pull the applied material in one piece from the skin. Alternatively, and preferably, the epilatory composition is supplied in the form of strips, sandwiched between sheets, for example of cellophane, or paper or another non-woven material. In use, one sheet is removed from a strip of epilatory composition and that strip is then applied to the skin with the remaining sheet uppermost. The end of that sheet is grasped and pulled sharply, to remove the strip of epilatory composition from the skin, along with hairs with which it is in contact.

Because the epilatory composition does not flow even under very warm ambient conditions it may be applied to a sheet during manufacture so as to cover a larger area of the sheet, than has been achieved, with prior epilatory compositions. Preferably it covers at least 60% of the area of the sheet, more preferably at least 80%, most preferably at least 90%.

In accordance with a further aspect there is provided an epilatory product, comprising epilatory strips formed of an epilatory composition as defined herein, the epilatory strips being sandwiched between sheets which are peelable from the strips.

In accordance with a further aspect there is provided a method of epilation, using a composition or product of the invention.

The invention will now be further described, by way of example.

**Example 1**

A composition was made with the following ingredients.

| Ingredients | % wt/wt |
|---|---|
| DERTOLINE RC | 60.8 |
| GRANOLITE 150 | 34.2 |
| ELWAX 40W | 2 |

(continued)

| Ingredients | % wt/wt |
|---|---|
| HDK N20 | 3 |
| DERTOLINE RC is a rosin esterified with glycerol, and is available from DRT. GRANOLITE 150N is a rosin esterified with triethylene glycol, and is available from DRT. ELWAX 40W is an ethyl vinyl acetate, available from Du Pont. HDK N20 is a fumed silica powder available from Wacker. | |

The DERTOLINE was preheated to 60°C. The GRANOLITE and ELWAX were added to the vessel. The temperature was raised to 70°C while mixing took place. HDK N20 was added and homogenised with a high speed turbine, at a speed of 1100 rpm.

The resulting product, at ambient temperature, had the appearance of a viscous liquid-gel. The gel point at 50°C (G' = G") was at the frequency ($\omega$) 2 rad/s and at this temperature G' exceeded G" at frequencies below 2 rad/s, as measured using the SR rheometer, mentioned above.

**Example 2**

| Ingredients | % wt/wt |
|---|---|
| DERTOLINE RC | 70.6 |
| GRANOLITE 150 | 24.4 |
| Thick mineral oil (paraffin oil) | 2.0 |
| HDK N20 | 2.5 |
| Perfume | 0.5 |
| Dye | 0.5 |
| Anti-oxidant | 0.5 |

The DERTOLINE was preheated to 60°C. The GRANOLITE and thick mineral oil were added, and the materials heated to 70°C with mixing. The HDK N20 was added, and homogenised with a high speed turbine, operated at a speed in excess of 1000 rpm. The dye, perfume and anti-oxidant were introduced after cooling to 40°C.

The resulting epilatory composition was a viscous liquid-gel. Its viscosity (at 35°C, shear rate $10s^{-1}$, tool : parallel plate of 25 mm) was 284 Pa.s. Its gel point at 50°C (G' = G") was at the frequency ($\omega$) 1.95 rad/s and at this temperature G' exceeded G" at frequencies below 1.95 rad/s, as measured using the SR rheometer.

**Example 3**

Isothermal tests were performed on various cold epilatory composition containing different amounts of fumed silica to evaluate the impact of the fumed silica on the gel formation.

| Ingredients | % | % | % | % | % |
|---|---|---|---|---|---|
| Resin base | 95 | 96 | 97 | 98 | 99 |

(continued)

| Ingredients | % | % | % | % | % |
|---|---|---|---|---|---|
| AEROSIL 200 | 5 | 4 | 3 | 2 | 1 |

Resin base:

| Ingredients | % |
|---|---|
| DERTOLINE G5 | 30 |
| GRANOLITE 150N | 70 |
| AEROSIL 200 is another fumed silica product, very similar to HDK N20, and available from Degussa. DERTOLINE G5 is another grade of rosin esterified with glycerol. | |

The epilatory composition was provided as a piece 60 mm x 120 mm x 1 mm between two cellophane sheets, themselves of area 110 mm x 180 mm.

Strips were placed at different temperatures, namely 20°C, 40°C and 50°C and the time required for the epilatory composition to leak out through or past the cellophane sheet was measured.

The results were as follows:

| % fumed silica | 20°C | 40°C | 50°C |
|---|---|---|---|
| 0% | No leakage | Leakage after 24 hrs | Leakage after 12 hrs |
| 1% | No leakage | Leakage after 2 days | Leakage after 2 days |
| 2% | No leakage | Leakage after 4 days | Leakage after 3 days |
| 3% | No leakage | No leakage | No leakage |
| 4% | No leakage | No leakage | No leakage |
| 5% | No leakage | No leakage | No leakage |
| A "no leakage" result means there was no leakage over a 3 month period. No leakage after 3 months represents an excellent result, especially at 50°C. | | | |

**Example 4**

A sugar-based epilatory material was prepared using the following ingredients:

| Ingredients | % |
|---|---|
| Sugar (granulated white, food grade) | 75.7 |
| Citric acid monohydrate (neat) | 1.3 |
| Water | 13.5 |
| Potassium hydroxide (50% in water) | 1.5 |
| HDK N20 | 8.0 |

The process was as follows.

In a vessel citric acid and water were mixed at 60°C. Once they formed a solution white granular sugar was added under constant stirring. The temperature was raised to 120°C. After 10 minutes at this temperature, the mixture was allowed to cool to 60°C. Then the HDK N20 was added and the composition homogenised using a high speed turbine at 1000 rpm. Finally the potassium hydroxide was added under stirring. The resulting product at ambient temperature had the appearance of a viscous gel. Its gel point at 25°C (G'=G") was at the frequency 20 rad/s. At 25°C G' exceeded G" at frequencies below 20 rad/s as measured using the SR Rheometer.

### Example 5

An epilatory material was prepared, similar to that of Example 1 and blended in the same way, but with GRANOLITE 150N, a rosinous material, replaced by ATDAC LVE, a hydrocarbon resin based on aliphatic monomers of petroleum origin, from Hercules. The ingredients were as follows:

| Ingredients | % |
| --- | --- |
| Dertoline RC | 59 |
| ATDAC LVE | 34 |
| ELWAX 40W | 2 |
| HDK N20 | 5 |

The resulting epilatory composition at ambient temperature had the appearance of a viscous liquid-gel. Its gel point at 50°C was at the frequency 5 rad/s.

### Claims

1. An epilatory composition comprising a rosinous or sugar-based matrix material in admixture with particulate fumed silica.

2. An epilatory material according to claim 1, wherein the particulate material has particles of mean diameter 1-200 mm.

3. An epilatory composition according to claim 1 or 2, wherein the particulate fumed silica supplies at least 1% of the weight of the epilatory composition.

4. An epilatory composition according to any preceding claim, wherein the particulate fumed silica supplies up to 40% of the weight of the epilatory composition.

5. An epilatory composition according to any preceding claim, wherein at least 60% of the weight of the epilatory composition is provided by rosinous material.

6. An epilatory composition according to any preceding claim, and comprising a rosinous matrix material, wherein the sheets are shape stable, when not under applied stress, for a period of 6 months at all temperatures in the range 20-50°C.

7. An epilatory product comprising epilatory strips formed of an epilatory composition according to any preceding claim, the epilatory strips being sandwiched between sheets which are peelable from the strips.

8. A method of epilation, employing an epilatory composition according to any of claims 1 to 6 or an epilatory product according to claim 7.

### Patentansprüche

1. Enthaarungszusammensetzung, umfassend ein harzartiges oder auf Zukker-Basis vorliegendes Matrixmaterial in Gemisch mit teilchenförmiger pyrogener Kieselsäure.

**2.** Enthaarungsmaterial nach Anspruch 1, wobei das teilchenförmige Material Teilchen mit einem mittleren Durchmesser von 1-200 mm aufweist.

**3.** Enthaarungszusammensetzung nach Anspruch 1 oder 2, wobei die teilchenförmige pyrogener Kieselsäure mindestens 1 Gew.-% der Enthaarungszusammensetzung liefert.

**4.** Enthaarungszusammensetzung nach einem der vorangehenden Ansprüche, wobei die teilchenförmige pyrogener Kieselsäure bis zu 40 Gew.-% der Enthaarungszusammensetzung liefert.

**5.** Enthaarungszusammensetzung nach einem der vorangehenden Ansprüche, wobei mindestens 60 Gew.-% der Enthaarungszusammensetzung durch ein harzartiges Material bereitgestellt sind.

**6.** Enthaarungszusammensetzung nach einem der vorangehenden Ansprüche und umfassend ein harzartiges Matrixmaterial, wobei die Lagen über einen Zeitraum von 6 Monaten bei allen Temperaturen im Bereich von 20-50°C formstabil sind, wenn sie nicht unter Beanspruchung stehen.

**7.** Enthaarungsprodukt, umfassend Enthaarungsstreifen, die aus einer Enthaarungszusammensetzung nach einem der vorangehenden Ansprüche gebildet sind, wobei die Enthaarungsstreifen zwischen Lagen eingepasst sind, die von den Streifen abgezogen werden können.

**8.** Verfahren zum Enthaaren unter Einsatz einer Enthaarungszusammensetzung nach einem der Ansprüche 1 bis 6 oder eines Enthaarungsprodukts nach Anspruch 7.

**Revendications**

**1.** Composition épilatoire comprenant une matière matricielle résineuse ou à base de sucre mélangée avec une poudre de silice ultrafine particulaire.

**2.** Matière épilatoire selon la revendication 1, dans laquelle la matière particulaire a des particules ayant un diamètre moyen de 1 à 200 mm.

**3.** Composition épilatoire selon la revendication 1 ou 2, dans laquelle la poudre de silice ultrafine particulaire fournit au moins 1 % du poids de la composition épilatoire.

**4.** Composition épilatoire selon l'une quelconque des revendications précédentes, dans laquelle la poudre de silice ultrafine particulaire fournit jusqu'à 40 % du poids de la composition épilatoire.

**5.** Composition épilatoire selon l'une quelconque des revendications précédentes, dans laquelle au moins 60 % du poids de la composition épilatoire sont fournis par une matière résineuse.

**6.** Composition épilatoire selon l'une quelconque des revendications précédentes, et comprenant une matière matricielle résineuse, dans laquelle les feuilles ont des formes stables, quand elles ne subissent pas de contrainte appliquée, pendant une période de 6 mois à toutes les températures dans la plage de 20 à 50°C.

**7.** Produit épilatoire comprenant des bandes épilatoires formées d'une composition épilatoire selon l'une quelconque des revendications précédentes, les bandes épilatoires étant prises en sandwich entre des feuilles qui peuvent être décollées des bandes.

**8.** Procédé d'épilation employant une composition épilatoire selon l'une quelconque des revendications 1 à 6 ou un produit épilatoire selon la revendication 7.